# EUROPEAN PATENT SPECIFICATION

(11) **EP 2 651 392 B1**
(45) Date of publication and mention of the grant of the patent: **19.08.2015**
(21) Application number: 11799845.0
(22) Date of filing: 28.11.2011
(51) Int. Cl.: A61K 9/00, A61K 9/20, A61K 31/192

(54) **SOLID COMPOSITIONS CONTAINING FLURBIPROFEN, THE PROCESSES FOR THEIR PREPARATION AND THEIR USE.**
FESTE ZUSAMMENSETZUNGEN MIT FLURBIPROFEN, VERFAHREN ZU IHRER HERSTELLUNG UND IHRE VERWENDUNG
COMPOSITIONS SOLIDES CONTENANT DU FLURBIPROFÈNE, LEURS PROCÉDÉS DE PRÉPARATION ET D'UTILISATION

(30) Priority: 13.12.2010 IT BO20100731
(43) Date of publication of application: 23.10.2013
(73) Proprietor: ALFA WASSERMANN S.p.A., 65020 Alanno (PE) (IT)
(72) Inventor: MAFFEI, Paola, I-40133 Bologna (IT); FEDERICI, Mascia, I-40133 Bologna (IT); LAURO, Vittoria, I-40133 Bologna (IT); MASCAGNI, Marco, I-40133 Bologna (IT); VISCOMI, Giuseppe Claudio, I-40133 Bologna (IT)
(74) Representative: Hiebl, Inge Elisabeth
(86) International application number: PCT/IB2011/055320
(87) International publication number: WO 2012/080882

(56) References cited:
- EP-B1- 0 983 059
- WO-A1-02/098387
- WO-A2-98/42310

## Description

### Object of the invention

This invention refers to solid compositions for the preparation of oral dosage forms comprising flurbiprofen (FP), processes for their manufacture and their use as medicine.

The solid compositions, comprising FP in association with pharmaceutically acceptable excipients, are particularly suitable for the preparation of oral dosage forms, such as for example, lozenges, which are suitable for use in the treatment of inflammation of the oral cavity, being characterized by good tolerability, stability, and palatability.

### Background of the invention

The invention can be applied to the acid derivatives of non steroidal anti-inflammatory drug (NSAIDs), such as for example, ibuprofen, naproxen, fenoprofen, ketoprofen, indoprofen, carpofen, miroprofen, tiaxoprofen, alminoprofen, tiaprofenic acid and flurbiprofen.

The main mechanism action of nonsteroidal anti-inflammatory drugs is the inhibition of cyclo-oxygenase (COX) activity, with a consequent reduction of prostaglandin synthesis.

COXs convert arachidonic acid into prostaglandin H2 (PGH2), which is then metabolized in different prostanoids according to the kind of cells or tissue.

Two isoforms of COX are identified: COX1, constitutive and expressed in many tissues and COX 2, generally induced by pro-inflammatory stimuli.

With a reference to the present invention, the preferred members of the group of active ingredients belonging to the propionic acid derivatives, include flurbiprofen, naproxen, ketoprofen, ibuprofen and, more particularly, flurbiprofen.

Flurbiprofen, 2 - (2-fluoro-4-phenyl-phenyl) propionic acid, hereinafter FP, is well known for its anti-inflammatory, antipyretic and analgesic activity, for the treatment of pain, inflammation and fever, resulting for example from arthritis, osteoarthritis, ankylosing spondylitis, post-operative pain, postpartum pain and skin wounds. FP molecule exists in two enantiomeric forms and the term FP is understood here to mean both the mixture of two enantiomers in any proportion, including the racemic form and the two enantiomers individually.

FP can exist in the form of pharmaceutically acceptable salts or in the form of ester derivatives, which are more water soluble than the acid form.

Pharmaceutical compositions containing FP are known and commercially available in different dosage forms, such as for example, tablets, lozenges, mouthwashes, skin patches and spray solutions. In particular, the formulations in tablets, mouthwash and spray solutions are useful for the treatment of inflammations of the oralpharyngeal cavity, releasing useful therapeutic amounts of FP in the oralpharyngeal cavity.

Although NSAIDs are used for symptoms associated with inflammation of the oral cavity, they cause an unpleasant burning sensation that is not acceptable to the patients being treated. Consequently, processes for the production of tablets or lozanges containing non-steroidal anti-inflammatory drug have all been studied with the aim of overcoming the problem of patient acceptability. Many solid formulations have been studied over the years with techniques and ingredients available to experts in the field, to find effective formulations that can release the active ingredient and with an improved patient acceptability.

EP 0862424 describes a process for the production of solid dosage forms, for example lozenges, tablets containing 2.5 to 20 mg of FP. The process includes the addition of solid FP to a mass of sugars or alcohols sugar melted, for example sorbitol, in a mixture with glucose. The mixture is then distributed for the production of the lozenges. Dosage forms produced in this way provide relief from symptoms of inflammation in the oral cavity, without producing an unacceptable burning sensation. These dosage forms may contain additional ingredients that are added to the melted mixture before or after the addition of FP or incorporated into granules.

WO 2006/092569 describes a process for the production of lozenges containing FP, which provides the formation of a liquid mixture constituted by one or more solvents selected from water, alcohol, polyols or polyethers containing FP, the melting of a mixture of sugars comprising glucose and the union of the two mixtures. The process of this invention overcomes the problem of EP 0862424 in which the addition of some solid excipients, such as the flavorings, to the melted mass of sugars, leads to unstable lozenges. Ingredients such as flavoring, stabilizer or sweetener agents are added to the mass of melted sugars or to the FP solution. The total weight of the lozenges produced by the process described in WO 2006/092569 is greater than 2 g.

EP 0983059 describes a process that involves the formation of granules containing FP and their subsequent mixing to a mass of melted sugars. The total weight of the lozenges produced by the process described in this invention is greater than 2 g. The composition obtained with the process of EP 0983059 comprises a percentage of sugar of 99% by weight.

WO02/098387 A1 describes pharmaceutical compositions comprising a low melting point non steroidal-anti-inflammatory drug which also FP is comprised, in form of granules and an organic acid. The granules are obtained heating the non steroidal-anti-inflammatory drug up its melting point; the molten mixture is cooled at temperature below its normal melting point to obtain the granules, which are then mixed with selected extragranular excipients, including an organic acid. In the working examples FP has a percentage of 2.5% and 10% in respect to the total weight of the composition.

WO98/42310 describes pharmaceutical composition in form of tablets of FP, which comprise a disintegrant, a compressible carrier material, selected from a sugar component, a starch component and an alkaline earth metal component, characterized in that the compressible carrier material further comprises microcrystalline cellulose. The process described in WO98/42310 comprises forming of granules of FP and compressible carrier material in the presence of an inert solvent.

EP 1992333 A1 describes pharmaceutical composition comprising FP in combination with an alpha-2 adrenergic receptor or a gamma-aminobutiric acid receptor agonist, and the amount of FP is comprised between 100 mg and 500 mg.

WO2008/095186A1 describes a process to obtain enantiomer R (-) of FP. The obtained product comprises small quantities of related impurities derived from the production process and one of them is the enantiomer S(+) of FP. This document also describes pharmaceutical compositions in the form of tablets or lozenges comprising a quantity of 100 mg and 400 mg of enantiomer R(-) of FP in the presence of small quantities of enantiomer S (+).

Therefore there is a need to provide efficacious compositions in solid forms comprising FP which are not irritating for the mucosa of the oral cavity and, at the same time, have a pleasant and acceptable taste, an improved palatability and patient acceptability. There is the further need to provide a simple, reproducible and scalable process for production in industrial facilities to obtain compositions stable in the time for the treatment of inflammatory diseases of the oral cavity, including the oral-pharyngeal cavity.

The present invention concerns a solid pharmaceutical composition for the production of oral dosage forms, in the form of lozenges, characterized in that it is glucose free and it comprises:
- flurbiprofen, or a pharmaceutically acceptable salt or an ester thereof, in an amount from between 2.5 to 20.0 mg, and pharmaceutically acceptable excipients, according to the following percentages:
- Flurbiprofen: 0.25-1.0% (w/w)
- Lubricant agent(s):glycerol dibehenate, 1.0-5.0% (w/w)
- Sweetener agent(s): acesulfame potassium, 0.5-5.0% (w/w)
- Diluent agent(s): mannitol, 84.0-98.0% (w/w)
- Binder agent(s):copovidone, 0.25-5.0% (w/w)

One aspect of the said invention is characterized by the fact that the selected excipients and the relative ratios, ensure good compressibility and the obtainment of stable lozenges.

Another important aspect of the invention, are compositions comprising FP without sugars, such as glucose. This composition is therefore particularly suitable for administration in diabetic patients.

Another aspect of the said invention is a process for the production of solid dosage forms, preferably lozenges, that includes a single step, avoiding problems of degradation of active ingredient and excipients.

Another aspect of the invention is that the total weight of the lozenges produced by the process described is minor than 2 g, preferably minor than 1.5 g.

Another aspect of the composition is the fact that the composition comprising FP provides solid dosage forms, preferably lozenges, that are safe, well tolerated, effective, with local activity in the oropharyngeal tract, and systemic activity in patients with problems of inflammation, fever and/or joint pain.

### Description of the invention

The present invention describes solid compositions comprising FP, for the preparation of solid dosage forms for oral administration, such as lozenges, stable at room temperature and which, in particular, have a good taste acceptable to the palate, without causing an unpleasant burning sensation or irritation and are useful in patients suffering of inflammation and pain of oral cavity.

The solid dosage forms for oral administration comprise a quantity of FP comprised from between 2.5 to 20.0 mg, more preferably a quantity comprised from between 5.0 to 15.0 mg. The total weight of the solid composition in form of lozenges is minor than 2.0 g and preferably minor than 1.5 g.

The amounts and the percentages of active ingredient specified in the present invention refer to free FP also when it is employed in the form of its pharmaceutically acceptable salts and its esters.

According to the invention, solid compositions comprise together with the active ingredient FP a number of auxiliary agents, appropriately chosen, in particular, one or more lubricant and /or glidant agent(s), one or more sweetener agent(s), one or more diluent agent(s), one or more binder agent(s). According to the invention, in addition to these essential agents, the composition can optionally contain one or more non-essential ingredients such as, for example, flavoring agents, opacifier agents, buffering agents, antibacterial or antiseptic agents.

The lubricant agent suitable for the preparation of the composition of the invention is glycerol dibehenate.

The diluent agent suitable for the preparation of the composition of the invention is mannitol.

The binder agent suitable for the preparation of the invention is copovidone (polyvinylpyrrolidone).

The sweetener agent suitable for the preparation of the composition of the invention is acesulfame potassium.

The composition can further comprise bioadhesive agents which are able to increase the contact of the active principle with the oropharingeal mucosa. The bioadhesive agents (s) suitable for the preparation of the composition object of the present invention is (are) chosen from the group comprising natural agents such as pectins, zeins, casein, gelatin, albumin, collagen, kitosan, oligosaccharides and polysaccharides such as, for example, dextran, polysaccharides from tamarind seeds, xanthan gum, Arabic gum, hyaluronic acid, alginic acid, sodium alginate, or synthetic agents such as polyamides, polycarbonates, polyalkylenes, polyalkylene glycols, polyalkylene oxides, polyalkylene terephthalates, polyvinyl alcohol, polyvinyl ethers, polyvinyl esters, polyvinyl pyrrolidone, polysiloxanes, polyurethanes, polystyrenes, polymers of acrylic acid and methacrylate esters, copolymer of methacrylic acid-ethyl acrylate, polyactides, polybarbituric acids, polyanhydrides, polyorthoesters and mixtures thereof. Other useful polymers are methyl cellulose, ethyl cellulose, hydroxy propyl cellulose, hydroxy butyl methylcellulose, cellulose acetate, cellulose propionate, cellulose acetate butyrate, carboxy methyl cellulose, cellulose triacetate, cellulose sulfate sodium salt, polymethyl methacrylate, poly-isopropyl methacrylate, poly-isobutyl acrylate, poly(octadecyl acrylate), polypropylene, polyethylene glycol, polyethylene oxide, polyethylene terephthalate, polyvinyl acetate, polyvinyl chloride, polystyrene, polyvinyl pyrrolidone, polyvinyl phenol and mixtures thereof.

According to the invention, the solid composition comprises a percentage of the active ingredient FP, and of the lubricant, sweetener, diluent and binder agent(s) in respect to their total weight, which varies according to the scheme reported in Table 1.

**Table 1**

| **Component** | **Percentage (%) by weight** |
|---|---|
| Flurbiprofen | 0.25 - 1.0 |
| Lubricant agent(s) | 1.0 - 5.0 |
| Sweetener agent(s) | 0.5 - 5.0 |
| Diluent agent(s) | 84.0 - 98.0 |
| Binder agent(s) | 0.25 - 5.0 |

The binder agent is copovidone.

One or more flavoring agent(s), such as for example a natural flavor like mint, may optionally be comprised as additional ingredient.

The composition can also comprise one or more antioxidant, opacifier or buffering agents.

A preferred aspect of this invention consists in a solid composition for oral administration, characterized in that the dosage form obtained, comprises from between 2.5 to 100.0 mg of the active ingredient FP, lubricant agent, sweetener agent, diluent agent and binder agent as reported in Table 2 below, wherein the percentages by weight are referred to the total weight of the active ingredient, the lubricant, sweetener, diluent and binder agents, for a single dosage.

**Table 2**

| **Component** | **Percentage (%) by weight** |
|---|---|
| Flurbiprofen | 0.25 - 1.0 |
| Glycerol dibehenate | 1.0 - 5.0 |
| Acesulfame potassium | 0.5 - 5.0 |
| Mannitol | 84.0 - 98.0 |
| Copovidone | 0.25 - 5.0 |

A more preferred aspect of invention consists in a solid composition for the preparation of lozenges which comprises a quantity of FP from 5 mg to 15 mg and the percentage of FP ranges from 0.25% to 1.0%, in respect to the total weight of the composition.

To this mixture, an amount of flavoring agent such as balsamic mint can optionally be added, ranging from between 2.0 to 10.0% in respect to the weight of the composition specified in Table 2.

A solid composition for oral administration particularly preferred according to the said invention, comprises as essential components, the components listed in Table 3 of Example 1, in the amount and percentage by weight specified for a single dosage form.

The lozenges are obtained by a process in which the excipients are sieved and then mixed in a V-blender.

The mixture is then divided and the lozenges are obtained by a direct compression process that reduces the number of step and allows better control of the process. Moreover, the process does not involve wetting or drying or heating, which can lead to uncontrolled degradations of the active ingredient and excipients.

The rounded shape of the lozenges, with a central depression, ensures maximum contact with saliva and is suitable for releasing the FP content when sucked by the patient. Moreover, the rounded shape associated with the low weight of the lozenges render the composition particularly well accepted by the patients.

Example 1 describes the solid composition comprising FP and the preparation thereof by means of a process that involves the mixing in a V-blender of the active ingredient with one or more lubricants, sweeteners, diluents and binders (essential agents), wherein one or more non-essential ingredients such as those mentioned above, can be optionally added.

The rounded lozenges so obtained are stable for long periods at temperatures up to 25°C for at least 3 years.

The lozenges prepared as described in Example 1 release FP when dissolved in the mouth and the dissolution time has been determined as described in Example 2, in which the lozenges were immersed in a solution with a pH value between 6.0 and 7.6, in order to mimic the physiological conditions of the oral cavity, at a temperature ranging between 35°C and 40°C. The dissolution time, obtained on an average of six lozenges, is resulted to be more than twenty minutes, demonstrating that the formulation of the present invention releases the FP when dissolved in the mouth.

The solid composition comprising FP, object of the present invention, has been tested in a clinical trial in healthy volunteers to evaluate safety, tolerability and acceptability.

The clinical trial has demonstrated the safety and tolerability of the composition in lozenges comprising FP, which, acting particularly at the local level, i.e. on the oropharyngeal mucosa, has relieved patients suffering from inflammation and pain of the oral cavity.

The quantity of FP not absorbed by the oropharyngeal mucosa is ingested by the patient and enters the bloodstream, also providing an analgesic, anti-inflammatory and antipyretic effect.

The safety and tolerability of the solid composition in form of lozenges comprising FP has been assessed by the clinical trial described in Example 3 and Example 4, in which twenty-six healthy volunteers received a lozenge comprising 8.75 mg of FP, four times a day for a period of five days and one lozenge on the sixth day.

The onset of local clinical signs such as irritation, erythema, edema, petechial hemorrhage or lesions of the oral mucosa and vital parameters such as changes in heart rate, blood pressure and respiratory rate were evaluated throughout the treatment period. No particular signs and no changes have been observed in comparison to the data reported prior to the treatment, demonstrating that the composition in form of lozenges comprising FP is well tolerated and therefore safe.

No one volunteer has abandoned the clinical trial or has reported significant problems, demonstrating that the pharmaceutical composition of the present invention is well tolerated even after repeated administrations.

No one volunteer complained about a possible scarce acceptability of the composition with regard to its palatability.

A pharmacokinetic study has been carried out to confirm that the developed composition is able to release the active ingredient on the anatomic site interested by the inflammatory process (oropharingeal cavity), and its result is that the composition exerts not only a local analgesic effect, but also a systemic effect.

In the clinical trial reported in Example 5, twenty-six healthy volunteers have received a lozenge comprising FP, prepared as described in Example 1, four times a day, with the administration of one lozenge every four to six hours for five days and the administration of one lozenge on the sixth day.

Plasma concentrations was determined in the morning of the day before the first administration and in the morning of the days 1, 4, 5, and 6 after the administration. The plasma concentration has been determined after 1.5, 3 and 5 hours since the last treatment.

The value of the plasma concentration (Cₘₐₓ) of FP has been determined by means of an HPLC chromatographic method, in the presence of a fluorimetric detector; also the time corresponding to the maximum plasma concentration (Tₘₐₓ), the area subtended to the curve (AUC₀₋₂₄) of the plasma concentrations during treatment and the removal time (T_{1/2}) have been determined.

The obtained plasma concentration reaches its maximum value comprised between 1.5 and 2.5 mg/ml after 1.5 hours since the last administration on the sixth day, confirming that the new formulation is suitable to be used as a local analgesic and anti-inflammatory of the oropharyngeal mucosa without however reaching high plasma concentrations of FP that could cause adverse effects, such as, gastro-intestinal bleeding.

In addition, the pharmacokinetic profile shows low FP levels in the plasma, making this formulation safe even after repeated administration.

The removal time (T_{1/2}), 1.5 hours after the final administration was found to be comprised between 1 and 2.5 hours, the area subtended to the curve of the plasma concentrations between 0 and 24 h (AUC₀₋₂₄ₕ) was found to be between 1.5 and 8.0µg/ml/h and the area subtended to the plasma concentration curve between 0 and infinite time, (AUC_{0-∞}) was found to be between 0 and 31 µg·ml/h.

The FP systemic concentrations turned out to be very low, demonstrating a low risk of adverse events, usually provoked by NSAIDs, for example gastrointestinal ones, but with systemic activities.

In Table 5 of Example 5, are reported the FP plasma concentration values in subjects who received the lozenges during the treatment period demonstrating that the FP does not accumulate in the blood and that these compositions can be used at FP concentrations higher than those given in the examples, with a major number of administrations per day. The composition comprising FP of the present invention can be administered from one to four times a day and more, without any negative effect.

In addition, the compositions may contain vitamins, amino acids, other natural extracts or mineral compounds, buffering, glidant and antioxidant agents.

When other active ingredients are combined with the NSAID and in particular with FP, the dosage is not limited to a specific component, but to the mixture of several NSAIDs.

In addition, the NSAIDs in association to the FP can be prepared and administered in the same composition described in the invention, or prepared and administered separately without any limiting effect.

The compositions of the present invention can therefore be used at FP concentrations greater than those reported in the examples, with a greater total number of administrations per day, having shown that the active ingredient does not accumulate in the bloodstream and does not show any toxic effects.

The described compositions may include gastro-protective, antiemetic, anti-ulcer, anti-stress or anti-anxiety agents, anti-acid drugs, gastric secretion inhibitors, such as anti-cholinergic drugs, muscarine receptor inhibitors, antibacterials, antitussives, mucolytics and antihistamines.

The described examples, which demonstrate the invention, must not be intended as limiting of the invention.

### Example 1

### Preparation of a solid composition comprising FP.

The solid components, in the quantities reported in Table 3, for a single dosage unit, are sieved with a 0.8 mm mesh sieve and mixed in a V-blender.

**Table 3**

| **Component** | **Quantity (mg)** | **% (w/w)** |
|---|---|---|
| Flurbiprofen | 8.75 | 0.92 |
| Glycerol dibehenate | 20.00 | 2.09 |
| Acesulfame potassium | 8.00 | 0.84 |
| Mannitol | 913.25 | 95.64 |
| Copovidone (Vinyl pyrrolidone vinyl acetate copolymer) | 5.00 | 0.52 |

Before sieving, 45 mg of balsamic mint flavoring is added to the mixture. The mixture is compressed using a tabletting machine, equipped with a flat-shaped hole of 16 mm. The lozenges are then stored in a blister which is sealed by thermal treatment using a sheet of aluminum.

The hardness of the lozenges so obtained, determined in accordance with the European Pharmacopoeia Ed 6.0, page 279, is 16 Kp.

### Example 2

### Measure of the dissolution time of the solid composition comprising FP.

The dissolution time of the lozenges has been determined in accordance with the European Pharmacopoeia "Guidance on dissolution testing" Ed. 6 at pH 6.0 and pH 7.6 with some modifications.

The solution at pH 6.0 has been prepared by placing 250 ml of 0.2 M potassium dihydrogen phosphate solution in a 1000 mL volumetric flask and adding 28 ml of 0.2 M sodium hydroxide and enough water to reach 1000 ml.

The pH 7.6 solution was prepared by placing 250 ml of 0.2 M potassium diacid phosphate solution in a 1000 mL volumetric flask and adding 212 ml of 0.2 M sodium hydroxide and water to reach 1000 ml.

Six lozenges prepared as in Example 1 have been respectively placed in the two solutions, stirred continuously at 100 rpm at 35 ± 0.5°C. Samples were taken after 15, 30, 45, 60, 90 and 120 minutes.

The percentages of FP released at pH 6.0 and pH 7.6, reported in Table 4, have been determined by using HPLC method and are represented by the average values obtained from the analysis at pH 6.0 and pH 7.6. The HPLC method employed involves the use of a reverse phase column, LiChrospher 100RP8, having size 250 x 4.0 mm, with 5 µm particles. The elution of the FP occurs in isocratic conditions with a solution made up of acetonitrile-water in a 42/58 volume ratio, brought to pH 3 using 85% (v/v) ortho-phosphoric acid.

The method foresees a fluorimetric detection at 260-310 nm and FP was eluted with a retention time, RT, corresponding to 16.6 minutes.

**Table 4**

| **Time (min)** | **% FP (p/p) with pH 6.0** | **% FP (w/w) with pH 7.6** |
|---|---|---|
| 15 | 40.3 | 72.2 |
| 30 | 75.1 | 97.1 |
| 45 | 91.1 | 97.5 |
| 60 | 97.1 | 98.3 |
| 90 | 97.6 | 97.8 |
| 120 | 98.3 | 98.1 |

### Example 3

### Determination of the safety of the solid composition comprising FP.

Twenty-six persons, healthy volunteers, among whom sixteen males and ten females, aged between 18 and 55 years, received one lozenge comprising 8.75 mg of FP prepared as in Example 1, four times a day for five consecutive days and one lozenge on the sixth day.

No alteration was shown with regard to vital parameters such as blood pressure, heart rate, breathing frequency and body temperature during the objective physical examination and during instrumental evaluations such as electrocardiogram and laboratory analysis.

### Example 4

### Determination of the local clinical tolerability of the solid composition comprising FP.

Twenty-six persons, all healthy volunteers, among whom sixteen males and ten females, aged between 18 and 55 years, received one lozenge comprising 8.75 mg of FP prepared as in Example 1, four times a day for five consecutive days and one lozenge on the sixth day.

For the evaluation of local tolerability, the researcher had to evaluate the recurrence of irritation, erythema, edema, petechiae bleeding and buccal ulcers throughout the days of treatment, according to a scale of "none", "scarce", "moderate" and "severe". Local tolerability was found to be good, only a few volunteers reported scarce symptoms while receiving the lozenge, which then disappeared in less than an hour.

### Example 5

### Bioavailability of the solid composition comprising FP as oral administration.

Twenty-six people, healthy volunteers, among whom sixteen males and ten females, aged between 18 and 55 years, received one lozenge comprising 8.75 mg of FP prepared as in Example 1, four times a day for five consecutive days and one lozenge on the sixth day.

The plasma concentration was determined for each patient before the first dose and, successively, in the morning of the days 1, 4, 5, and 6 and after 1.5, 3 and 5 hours since the last treatment.

Each sample was moved into a test tube containing heparin and centrifuged, and the plasma was divided into 500 µl aliquots and freezed at -80°C.

The concentration of FP contained in the plasma is represented by the average of the values obtained by the analysis carried out with the validated HPLC method in by using a reverse phase column, Hypersil C8, having a size 150 x 4.6 mm, with 5 µm particles. The FP elution occurs under isocratic conditions with an aqueous solution containing ortho phosphoric acid at 0.4% (v/v) and acetonitrile, in the volume ratio, respectively, of 55% and 45%. The method foresees a fluorimetric detection at 260-310 nm and has detection limit for FP corresponding to 0.025 µg/ml. The following parameters were determined:
Cₘₐₓ: maximal FP plasma concentration observed in plasma.
Tₘₐₓ: time in wherein Cₘₐₓ is reached.
AUC: area subtended to the concentration-time curve, calculated by means of the linear trapezoidal rule.

The average results of FP plasma concentrations on days 1, 4, 5 and 6 are recorded in Table 5.

**Table 5**

| **Day (hours)** | **Time (hours)** | **FP average plasma concentrations of (µg/ml)** |
|---|---|---|
| 1 | 0 | 0.000 |
| 4 | 0 | 0.807 |
| 5 | 0 | 0.763 |
| 6 | 0 | 0.797 |

Table 6 reported the average values of C_{max,} relating to the sixth day after 1.5, 3 and 5 hours respectively, since the last administration on the sixth day.

**Table 6**

| **Hours after the last administration** | **FP average plasma concentrations of (µg/ml)** |
|---|---|
| 1.5 | 1.856 |
| 3.0 | 1.507 |
| 5.0 | 1.507 |

The maximum FP plasma concentration was determined by means of the validated HPLC chromatographic method using a reverse phase column, Hypersil C8, having size 4.4 x 150 mm, with of 5 µm particles. The elution of FP occurs in isocratic conditions with an aqueous solution containing orthophosphoric acid at 0.4% (v/v) and acetonitrile, in the volume ratio, respectively, of 55% and 45%. The method foresees a fluorimetric detection at 260-310 nm, which was characterized by detection limit of FP corresponding to 0.025 µg/ml.

The maximum plasma concentration was reached on the sixth day after the last administration and in Table 7 are reported the values obtained after the last administration.

**Table 7**

| **Cₘₐₓ (µg/ml)** | **Tₘₐₓ (hours)** | **AUC ₀₋₂₄ₕ (µg/ml·h)** | **AUC_{0-inf}**. **(µg/ml·h)** |
|---|---|---|---|
| 1.912 ± 1.47 | 1.674 ± 0.49 | 4.68 ± 2.97 | 14.28 ± 16.66 |

### Example 6

### Determination of the palatability after the administration of the solid composition containing FP.

All volunteers were asked to express an opinion about the taste during and after administration. No one of the twenty-six healthy volunteers receiving the solid composition described in Example 1 complained about the taste or refused to receive the successive administration.

## Claims

1. A solid pharmaceutical composition for the production of oral dosage forms, in the form of lozenges, **characterized in that** it is glucose free and it comprises:
- flurbiprofen, or a pharmaceutically acceptable salt or an ester thereof, in an amount from between 2.5 to 20.0 mg, and pharmaceutically acceptable excipients, according to the following percentages:
- Flurbiprofen: 0.25-1.0% (w/w)
- Lubricant agent(s):glycerol dibehenate, 1.0-5.0% (w/w)
- Sweetener agent(s): acesulfame potassium, 0.5-5.0% (w/w)
- Diluent agent(s): mannitol, 84.0-98.0% (w/w)
- Binder agent(s):copovidone, 0.25-5.0% (w/w).

2. A solid pharmaceutical composition according to claim 1 comprising an amount of flurbiprofen from 5.0 to 15.0 mg, for each dosage unit.

3. A solid pharmaceutical composition according to claim 2 comprising one or more additional agent(s) selected from flavoring agents, opacifier agents, buffering agents, antioxidant, antibacterial, and antiseptic agents.

4. A solid pharmaceutical composition according to claim 3 comprising flavorings, in particular mint flavor in an amount from 2.0% to 10.0% (w/w) in respect to the weight of the composition.

5. A pharmaceutical composition according to claim 4 **characterized in that** the amount of flurbiprofen for each dosage unit is comprised between 5.0 and 15.0 mg.

6. A pharmaceutical composition according to claim 1 comprising flurbiprofen, or a pharmaceutically acceptable salt or a ester thereof and pharmaceutically acceptable excipients in the following amounts for each dosage unit: flurbiprofen 8.75 mg, glycerol dibehenate 20.00 mg, acesulfame potassium 8.00 mg, mannitol 913.25 mg and copovidone 5.00 mg.

7. A pharmaceutical composition according to claim 6 comprising 45 mg of balsamic mint flavor.

8. A pharmaceutical composition according to claim 6 comprising a quantity of flurbiprofen from 0.25% to 1.0% (w/w) with a composition total weight minor than 2 g.

9. A pharmaceutical composition according to any one of claims 1 to 8 for use as medicament.

10. A pharmaceutical composition for the use according to claim 9 for use in the treatment of inflammations of the oral cavity.

11. A pharmaceutical composition for the use according to claim 10 further **characterized in that** flurbiprofen is administered to patients in a solid.

## Patentansprüche

1. Feste pharmazeutische Zusammensetzung für die Herstellung oraler Dosierungsformen, in der Form von Pastillen, **dadurch gekennzeichnet, dass** sie glucosefrei ist und dass sie umfasst:
- Flurbiprofen oder ein pharmazeutisch annehmbares Salz oder einen Ester davon in einer Menge von zwischen 2,5 und 20,0 mg und pharmazeutisch annehmbare Exzipientien entsprechend den folgenden Prozentgehalten:
- Flurbiprofen: 0,25-1,0% (G/G)
- Schmiermittel: Glycerindibehenat, 1,0-5,0% (G/G)
- Süßungsmittel: Acesulfam-Kalium, 0,5-5,0% (G/G)
- Verdünnungsmittel: Mannit, 84,0-98,0% (G/G)
- Bindemittel: Copovidon, 0,25-5,0% (G/G).

2. Feste pharmazeutische Zusammensetzung gemäß Anspruch 1, die eine Menge von Flurbiprofen von 5,0 bis 15,0 mg für jede Dosierungseinheit umfasst.

3. Feste pharmazeutische Zusammensetzung gemäß Anspruch 2, die ein oder mehrere zusätzliche(s) Mittel, ausgewählt aus Aromatisierungsmitteln, Trübungsmitteln, Puffermitteln, Antioxidans, antibakteriellen und antiseptischen Mitteln, umfasst.

4. Feste pharmazeutische Zusammensetzung gemäß Anspruch 3, die Aromatisierungsmittel, insbesondere Pfefferminzaroma, in einer Menge von 2,0% bis 10,0% (G/G), bezogen auf das Gewicht der Zusammensetzung, umfasst.

5. Pharmazeutische Zusammensetzung gemäß Anspruch 4, **dadurch gekennzeichnet, dass** die Menge von Flurbiprofen für jede Dosierungseinheit zwischen 5,0 und 15,0 mg liegt.

6. Pharmazeutische Zusammensetzung gemäß Anspruch 1, die Flurbiprofen oder ein pharmazeutisch annehmbares Salz oder einen Ester davon und pharmazeutisch annehmbare Exzipientien in folgenden Mengen für jede Dosierungseinheit umfasst: Flurbiprofen 8,75 mg, Glycerindibehenat 20,00 mg, Acesulfam-Kalium 8,00 mg, Mannit 913,25 mg und Copovidon 5,00 mg.

7. Pharmazeutische Zusammensetzung gemäß Anspruch 6, die 45 mg balsamartiges Pfefferminzaroma umfasst.

8. Pharmazeutische Zusammensetzung gemäß Anspruch 6, die eine Menge von Flurbiprofen von 0,25% bis 1,0% (G/G) bei einem Zusammensetzungsgesamtgewicht von weniger als 2 g umfasst.

9. Pharmazeutische Zusammensetzung gemäß einem der Ansprüche 1 bis 8 zur Verwendung als Medikament.

10. Pharmazeutische Zusammensetzung zur Verwendung gemäß Anspruch 9 zur Verwendung bei der Behandlung von Entzündungen der Mundhöhle.

11. Pharmazeutische Zusammensetzung zur Verwendung gemäß Anspruch 10, die außerdem **dadurch gekennzeichnet ist, dass** Flurbiprofen in einem Feststoff an Patienten verabreicht wird.

## Revendications

1. Composition pharmaceutique solide pour la production de formes de dosage posologiques orales, sous la forme de losanges, **caractérisé en ce qu'**elle est exempte de glucose et elle comprend:
- du flurbiprofène ou un sel pharmaceutiquement acceptable ou un ester de celui-ci en une quantité de 2,5 à 20,0 mg et des excipients pharmaceutiquement acceptables selon les pourcentages suivants:
- flurbiprofène: de 0,25 à 1,0% (p/p)
- agent(s) lubrifiant(s): dibéhénate de glycéryle: de 1,0 à 5,0% (p/p)
- agent(s) édulcorant(s): acésulfam de potassium: de 0,5 à 5,0% (p/p)
- agent(s) diluant(s): mannitol: de 84,0 à 98,0% (p/p)
- agent(s) diluant(s): copovidone: de 0,25 à 5,0% (p/p)

2. Composition pharmaceutique solide selon la revendication 1, comprenant une quantité de flurbiprofène de 5,0 à 15,0 mg, par unité de dosage.

3. Composition pharmaceutique solide selon la revendication 2, comprenant un ou plusieurs agent(s) aupplémentaire(s) choisi(s) parmi les agents aromatisants, les agents opacifiants, les agents tampons, les antioxydants, les agents antibactériens et les agents antiseptiques.

4. Composition pharmaceutique solide selon la revendication 3, comprenant des agents aromatisants, en particulier l'arôme de menthe, en une quantité de 2,0% à 10,0% (p/p) par rapport au poids de la composition.

5. Composition pharmaceutique selon la revendication 4, **caractérisé en ce que** la quantité de flurbiprofène est comprise entre 5,0 et 15,0 mg par unité de dosage.

6. Composition pharmaceutique selon la revendication 1, comprenant du flurbiprofène ou un sel pharmaceutiquement acceptable ou un ester de celui-ci dans les quantités suivantes par unité de dosage: flurbiprofène 8,75 mg, dibéhénate de glycéryle 20,00 mg, acésulfame de potassium 8,00 mg, mannitol 913,25 mg et copovidone 5,00 mg.

7. Composition pharmaceutique selon la revendication 6, comprenant 45 mg d'arôme de menthe balsamique.

8. Composition pharmaceutique selon la revendication 6, comprenant une quantité de flurbiprofène de 0,25% à 1,0% (p/p), le poids total de la composition étant inférieur à 2g.

9. Composition pharmaceutique selon l'une quelconque des revendications 1 à 8 pour l'utilisation en tant que médicament.

10. Composition pharmaceutique pour l'utilisation selon la revendication 9 pour l'utilisation dans le traitement des inflammations de la cavité buccale.

11. Composition pharmaceutique pour l'utilisation selon la revendication 10, **caractérisé en outre en ce que** le flurbiprofène est administré à un patient sous une forme solide.
